Europäisches Patentamt

European Patent Office

Office européen des brevets

(11) Publication number: **0 321 008 B1**

# EUROPEAN PATENT SPECIFICATION

(45) Date of publication of patent specification: **01.09.93**   (51) Int. Cl.5: **G01N 33/58**, G01N 33/532

(21) Application number: **88202597.6**

(22) Date of filing: **18.11.88**

The file contains technical information submitted after the application was filed and not included in this specification

(54) **Immunodetermination using non-metallic labels.**

(30) Priority: **19.11.87 NL 8702769**

(43) Date of publication of application:
**21.06.89 Bulletin 89/25**

(45) Publication of the grant of the patent:
**01.09.93 Bulletin 93/35**

(84) Designated Contracting States:
**AT BE CH DE ES FR GB GR IT LI LU NL SE**

(56) References cited:
**EP-A- 0 007 654        EP-A- 0 032 270**
**EP-A- 0 142 810        EP-A- 0 163 312**
**EP-A- 0 298 368        EP-A- 4 341 757**

**JOURNAL OF CHROMATOGRAPHY, vol. 376, 1986, pages 175-189, Elsevier Science Publishers B.V., Amsterdam, NL; T.C.J. GRIBNAU et al.: "Particle-labelled immunoassays: a review"**

(73) Proprietor: **H.B.T. HOLLAND BIOTECHNOLOGY B.V.**
**Niels Bohrweg 13**
**NL-2333 CA Leiden(NL)**

Proprietor: **Staat der Nederlanden ( Dienst Landbouwkundig Onderzoek(DLO))**
**Bornsesteeg 53**
**NL-6700 AB Wageningen(NL)**

(72) Inventor: **van Doorn, Albert Willem Jacob**
**Jacob Marislaan 60**
**NL-6813 JX Arnhem(NL)**
Inventor: **Wichers, Jan Herman**
**Tarthorst 703**
**NL-6708 JA Wageningen(NL)**
Inventor: **van Gelder, Wilhelmus Martinus Josef**
**M.L. Kingplantsoen 2**
**NL-6671 BP Zetten(NL)**

(74) Representative: **Smulders, Theodorus A.H.J., Ir. et al**
**Vereenigde Octrooibureaux Nieuwe Parklaan 97**
**NL-2587 BN 's-Gravenhage (NL)**

## Description

This invention relates to a method of determining in a test sample one or more components of the reaction between a specifically-binding protein and the corresponding bindable substance, using the mutual reactivity of such components and of at least one labelled component obtained by coupling or adsorbing particles of a sol of the label directly or indirectly to the component, comprising during, or after the completion of, the reaction, optionally after the separation of the bound and the free labelled component, determining in said test sample, or in one of the fractions obtained after separation, the presence and/or the quantity of the label by a method suitable for the purpose to obtain a qualitative or quantitative indication of the component to be determined.

The invention further relates to a method of preparing a labelled component of the reaction between a specific binding protein and the corresponding bindable substance by coupling or adsorbing particles of a sol of the label directly or indirectly to the component, and to a test kit for use in the determination of an immuno component in an aqueous test sample.

As used herein, the phrase "component of the reaction between a specific binding protein and the corresponding bindable substance" means substances, or parts thereof, such as receptor proteins and antigenic determinants, which may be present at the surface of cells, and immuno chemical substances, such as haptens, antigens, and antibodies, which may be present in aqueous mediums, in particular body fluids, such as blood plasma, serum, and the like, or culturing mediums of cells. The invention is accordingly concerned with a plurality of fields of histology, such as tissue and cell staining, in which immunochemical reactions take place, but also couplings may take place between the colloidal label and a non-immunochemical component, such as DNA and/or RNA, while furthermore couplings between the colloidal label and other macromolecular structures, such as enzymes, streptavidin, etc., are possible. In addition to these fields, the invention is also concerned with the field of immunoassay for, for example, diagnostic purposes (determination of antibodies, antigens or haptens in aqueous test samples). In the part of the specification which follows, the invention will be described in more detail with particular reference to the application of the invention to the field last mentioned, i.e., diagnostic immunoassays, but the invention should not be construed as being limited to such application, as it is equally applicable to histological and histochemical examination methods.

In EP-A-0007654, a survey is given of known irm unochemical methods in which the presence of a given immunological component is determined qualitatively and/or quantitatively, using the mutual reactions between such components, such as the reaction between antigen and the antibody against it. These known methods each have certain disadvantages or drawbacks which, according to the above European patent application can be removed by using in a method of the kind described in the opening paragraph a metal-labelled component, obtained by coupling or adsorbing the component directly or indirectly to particles of an aqueous dispersion of a metal or metal compound or of polymer nuclei coated with a metal or metal compound, with the particles having a size of 6-100 nm.

The metal-immunochemical technique described is not only more sensitive than the known radio- and enzyme-immuno techniques, but in addition renders it possible to demonstrate and determine more than one immunological component in the same test medium simultaneously by using different metal labels.

The metal sols may be of metals, or metal compounds, such as metal oxides, metal hydroxides or metal salts. As examples are mentioned the metals or metal compounds of gold, silver, iron, nickel, aluminium, chromium, lead, vanadium, mercury, manganese, and generally all those metals which can be readily demonstrated by means of known techniques.

Sols of metals are, for example, those of silver, gold and platinum. Sols of metal compounds are, for example, those of silver iodide, iron oxide, aluminium hydroxide, chromium hydroxide, vanadium oxide, iron hydroxide, manganese hydroxide and mercury sulfide.

Preferably, metals or metal compounds are used which do not occur in the test medium, and of these specifically those which can be demonstrated with a selected technique in as low a concentration as possible.

In EP-A-0032270, a survey is given of the possibilities of a qualitative and/or quantitative determination of an immunochemical component, in which one or more labelled components are used, which have been obtained by directly or indirectly coupling such a component or components to particles of an aqueous dispersion of a hydrophobic dye or pigment, or of polymeric nuclei coated with such a dye or pigment.

The earlier European Patent Application EP-A-0 298 368, which is part of the state of the art pursuant to Art. 54(3) EPC only, describes the use of colloidal non-metal particles, in particular elemental S, Se and Te, for labeling purposes.

Surprisingly it has now been found that certain non-metallic elements or inorganic compounds thereof which do not contain metallic elements can also be used as a label, and have certain advantages over and above the use of metal-containing labels.

The method according to the present invention is accordingly characterized by using for the labelling a sol of a non-metallic element selected from the group consisting of elemental P, elemental C and elemental Si or an inorganic compound of an element selected from the group consisting of S, Se, Te, P and Si, which inorganic compound does not contain metallic elements.

According to the invention, preferred sol particles are phosphorus, carbon and/or silicon. In another preferred embodiment, the sol particles used are inorganic compounds of selenium, tellurium, phosphorus, sulfur and/or silicon which do not contain metallic elements, such as silicon dioxide.

Such non-metallic sols can be prepared by a large number of known per se methods.

For the preparation of, for example, a phosphorus sol, reference is made to Berichte 37, 14 (1904); for a silicon sol to an article by G. Wegelin in Kolloid Z. 14, 65-69, 1914; and for a silicon oxide sol to J. of Colloid and Interface Science 26, 62-69, 1968.

For the preparation of some of the above mentioned sols, a new method was developed which uses borium hydride salts to reduce oxides of the non-metallic elements. The method is illustrated in the examples.

The sol particles of non-metallic elements carry a charge, which provides a stabilizing effect owing to mutual repulsion. By adding mainly strong electrolytes, the charge pattern is changed, which results in agglutination and flocculation. This can be prevented by enveloping the particles with macromolecules containing polar groups, such as proteins, polyethylene glycols, polymeric carbohydrates, polyvinyl alcohols, and the like. Suitable protective proteins are antigens, antibodies and anti-antibodies or immunochemically active fragments thereof, or haptens, coupled to immunochemically inert protective macromolecules, which directly results in immunocomponents labelled with sol particles of non-metallic elements.

It is preferable, however, not to use exclusively immunocomponents for enveloping the sol particles of non-metallic elements, because this does result in a stabilizing effect, but the immunochemical reactivity is less than could be expected, probably as a result of steric hindrance. Indeed, it has been found to be advantageous for the envelopment to be only partially effected with an immunocomponent, and for it to be completed with a different protective, but immunochemically inert material, such as inert proteins, for example albumin, a polyethylene glycol or another polar macromolecule. Another suitable enveloping material is protein A or related proteins possessing reactivity to the Fc portion of antibodies. After the envelopment of the sol particles of non-metallic elements by protein A, a further envelopment can be brought about with a desired antibody.

Another possibility is for the sol particles of non-metallic elements to be first enveloped with an inert hydrophilic polymer or copolymer, whereafter the immunological component is coupled to the enveloping material by adsorption or through covalent bonds.

The spheres obtained after envelopment may contain a single sol particle, but it is also possible for the polymer to envelop more than one sol particle.

The envelopment of the sol particles by the inert polymer can be effected in various ways, as described in the above EP-A-0007654.

The non-metallic sol particles to be used according to the present invention have a number of advantages over and above the metal sol particles described in the above patent application. Examples of such advantages are:

1. Carbon, for example, is less expensive than gold.

2. The method of preparing, for example, carbon sols is simpler and faster than of gold sols.

3. Gold sol particles are either small and monodisperse or large and not monodisperse (J.H.W. Leuvering, Thesis 1984), whereas this is not the case, or at least to a much lesser extent, with non-metallic sols (A.Watillon, J. Colloid and Interface Science 27 (3) 505-15 1968). These particles are monodisperse in virtually any size.

4. Sols of non-metals can have both a negative and a positive charge, depending on the method of preparation (Gmelins Handbuch Anorg.Chemie 1953 A pp. 26-27), which is not the case, for example, with gold sols.

5. For use as a label in sensitive test systems, such as agglutination (or inhibition of agglutination) methods, test strip methodology and spot/blot techniques, inorganic non-metallic colloidal particles having a relatively low specific mass offer the advantage of a higher solution stability at larger particle sizes, compared to inorganic metallic elements having much higher specific mass. Larger particle sizes are generally preferred as they improve the sensitivity of the text: detection of antibody-colloidal particle-antigen complexes formed in the immunochemical reaction is facilitated by a higher content of the detectable label in the complexes. In addition to the use of larger particles, test sensitivity may also be improved by coupling a large amount of immunochemically inert protein to the colloidal particles at the expense of the amount of specifically binding protein which is coupled to said particles. Such a measure will result in a reduction of the total binding capacity of the label conjugate.

In US-A-4,341,757, a method is described in which selenium is a part of a label. A selenium compound is first incorporated into an organic compound, whereafter the whole is bonded to an immunochemical component. When a complex is formed with a different specific immunochemical component, this complex is isolated, and the selenium in the complex is determined by atomic adsorption, for example.

In EP-A-0032270, a method is described in which use is made of a tracer consisting of a dispersion in water of a hydrophobic dye or pigment with an immunochemical component bonded thereto by means of a chemical reaction. The resulting complex is capable of reacting with a specific different immunochemical component. The dye or/and pigment used give the colour to the label system. This method is not specific, as it cannot be excluded in advance that the dye portion of the system - which by itself is an organic component - reacts with other products present in the test fluid.

The immunocomponents labelled with sol particles of non-metallic elements are used as reagents, commonly in combination with other reagents, for demonstrating and quantifying haptens, antigens and antibodies in an aqueous test medium, for which all sorts of immunochemical techniques, as are in use in radio immunoassays and enzyme immunoassays are suitable.

The invention accordingly also relates to test kits for use with such immunochemical techniques, and containing as the most important component an immuno component labelled with a non-metallic element or an organic compound thereof which does not contain metallic elements, consisting of a sol of a non-metallic element, the particles of which are either directly enveloped by the desired immunocomponent, or by an inert polymer to which the immunocomponent is coupled or adsorbed.

One of the conventional immunochemical techniques is the compatitive immunoassay, which can be used for demonstrating and determining an immunocomponent. For demonstrating, for example, a certain antigen, this method comprises contacting a test sample containing an unknown amount of antigen with either a pre-determined quantity of the antigen in question, labelled with a non-metallic element, and an insolubilized antibody against this antigen, or a pre-determined quantity of insolubilized antigen and an antibody directed against this antigen, labelled with a non-metallic element or compound thereof.

After completion of the reaction, the nature and/or quantity of the non-metallic element is determined in the bound or free fraction, which gives a qualitative and a quantitative indication, respectively, for the antigen to be determined. Mutatis mutandis, a similar method applies for determining other immunocomponents.

Other methods frequently being used are the so-called Sandwich techniques, which are also particularly suitable for the use of a component labelled with a non-metallic element according to the present invention. According to these techniques, an immunological component, for example, an antibody in case an antigen has to be determined, is insolubilized by coupling it to a solid carrier.

This solid carrier is, for example, the inner surface of the reaction vessel in which the immunochemical reaction is conducted. After a first incubation, possibly followed by a washing step, a second incubation is effected with an antibody labelled with a non-metallic element, whereafter the non-metallic element or a compound thereof is determined in the bound or the free phase.

The immunocomponents labelled with a non-metallic element also lend themselves well to the application in so-called homogeneous immunoassays, i.e., immunoassays in which a separation between the labelled immunological component bound in the immunochemical reaction and that which is still free is unnecessary.

Such assays have the advantage of being simple to perform, providing the desired information relatively fast, and lending themselves excellently for automation.

In the actual assay, for example, test sample (or standard solution) containing the antigen to be determined is incubated together with the labelled antibody in the wells of a microtiter plate. The immunochemical reaction between antigen and (labelled) antibody will result in agglutination. The thus induced agglutination of the particles in a sol of a non-metallic element is accompanied by a change in colour, which can be monitored e.g. spectrophotometrically or with the naked eye.

To determine small antigens, which in immunochemical respect are monovalent, use is made of an agglutination-inhibition reaction, which is based on the same principle.

In addition to the techniques mentioned above, there are countless other immunochemical techniques, in which the immunocomponent labelled with a non-metallic element can be used as a reagent. The present invention also makes it possible to demonstrate different haptens, antigens, antibodies, or combinations thereof in a test sample simultaneously, by using an immunocomponent as a reagent for each of the components to be demonstrated, which immunocomponent is labelled with a different sol particle of a non-metallic element.

The measurement of the nature and/or the concentration of the non-metallic element in a certain phase of the reaction mixture can be effected in countless, known per se manners. Examples are the colorimetric assay, in which use is made of the property that some sols of non-metallic elements are highly coloured dispersions: silicon = brown → yellow, phosphorus = fluorescent blue, which in addition change in colour upon physicochemical changes; the visual method, which sometimes is sufficient for qualitative assays, in view of the above fact that some sols of non-metallic elements are coloured; the use of flame emission spectrophotometry or any other plasma emission spectrophotometrical method, which enables simultaneous determination, and the highly sensitive method of flameless atomic absorption spectrophotometry.

In EP-A-0158746, a method is described, in which colloidal metal particles can be rendered (better) visible by using a physical developer. This physical developer is a silver containing compound. It has surprisingly been found that the visibility of colloidal particles consisting of non-metallic elements can also be enhanced by three to ten orders of magnitude. Even virtually colourless sols or compounds with sols, which therefore have extremely poor visibility, can thus be rendered visible, as described in the examples which follow.

The invention is illustrated in and by the following example.

Example : Preparation of "stained", monodisperse silicon dioxide sols

(a) Introduction

Colloidal $SiO_2$ particles are obtained by hydrolyzing tetraalkyl silicate esters in a mixture consisting of alcohol, water and ammonia. In this process, two kinds of reactions play a role:

1. hydrolysis of the tetraalkyl ester to form reactive silanols; in this process, ammonia acts as a catalyst.

2. transformation of the silanols formed into $SiO_2$, via a condensation reaction.

The diameter of the colloidal $SiO_2$ particles thus formed is mainly determined by the initial water and ammonia concentrations in the reaction mixture. The silicate sols obtained in the above manner are opalescent and exhibit a milky white colour.

"Staining" of colloidal silica particles became possible by allowing the hydrolysis of the tetraalkyl silicate esters and the subsequent condensation/polymerisation reactions to take place in a reaction vessel which in addition to the components specified contains a dye.

Such a dye should be well soluble in both alcohol and water. In connection with charge effects, dyes having alkaline properties lend themselves well to "staining" the (net) negatively charged $SiO_2$ particles.

If the dye selected has fluorescent properties, reading in the UV range of test results obtained by means of immunoassays in which "stained" $SiO_2$ particles function as a label is among the possibilities. The use of fluorescent $SiO_2$ labels in immunoassays will generally enhance the sensitivity of such test systems.

The use of $SiO_2$ labels "stained" by means of dyes which are clearly distinguishable from each other, and provided with antibodies with the required specificity offer the opportunity of demonstrating (or quantifying) different antigens (antigenic determinants) within one test sample fast and accurately.

(b) Preparation of a "stained" $SiO_2$ sol

In 862 ml absolute ethanol (Merck), 100 mg rhodamine isothiocyanate (RITC) (Sigma) is dissolved.

To this solution, 74 ml of a 25% aqueous $NH_3$ solution (Merck) is added while using a magnetic stirrer for mixing both solutions. Then 64 ml tetraethyl orthosilicate (Merck) is added quickly with vigorous stirring. The initial clear purplish/red solution becomes visibly opalescent after about 7 minutes.

After 120 minutes, the sol is purified by dialysis (by means of an artificial kidney). During the dialysis, the pH of the sol is decreased from pH 10.0 to pH 6.5. The dialysis is terminated as soon as the dialysate is found no longer to contain spectrophotometrically measurable

$$\left( A^{1 \ cm}_{560 \ nm} = 0 \right)$$

quantities of RITC.

There is thus formed an opalescent, purple $SiO_2$ sol contained in an aqueous medium.

The average diameter of the spherical $SiO_2$ particles is 156 nm ± 8 nm.

Prior to use, the sol is diluted to $A(\lambda = 560$ nm$) = 1.0$.

N.B.

I. Unprotected sol is rapidly flocculated under the influence of monovalent cations (e.g. Na + ). With the proviso of correct conditions (see Example 8 under (a)), physical adsorption of proteins to the colloidal silica particles sufficiently protects the sol from electrolyte-induced flocculation.

A suspension processed in accordance with the protocol, and to which, instead of 64 ml tetraethyl orthosilicate, 64 ml water has been added, exhibits no perceivable signs of flocculation after the addition of electrolyte.

The dialysis of this suspension leads to dramatic decoloration. All this indicates that colloidal RITC particles are not present and "stained" $SiO_2$ particles are present in the stained sol prepared by the above method.

II. The physical adsorption of protein to RITC-"stained" $SiO_2$ particles in alkaline medium (pH 9.5) theoretically offers the possibility for protein to be covalently bonded through an addition reaction involving the $-NH_2$ and/or the $-SH$ groups of the protein to the isothiocyanate group of the RITC, as far as these CNS groups would be accessible to the protein. In addition to the supposed bonding of protein to the $SiO_2$, there could possibly also be an undesirable bonding of protein with RITC. Under conditions of low(er) pH, the addition reaction referred to will be rendered more difficult.

With regard to the specificity of the reaction between an RITC-$SiO_2$-labelled immunoglobulin and a (protein) antigen, too, it is of importance to exclude an addition reaction.

(c) Adsorption of monoclonal mouse IgG to colloidal RITC/$SiO_2$

(All of the following operations are performed at room temperature, unless otherwise stated).

5 ml of the RITC/$SiO_2$ sol is adjusted to pH 7.0 by means of 0.1 M $K_2CO_3$.

50 $\mu$l mouse ascites is added with careful stirring to 5 ml neutralized RITC/$SiO_2$ sol. After 15 minutes incubation, the volume is made up with water to 15 ml.

The RITC/$SiO_2$-IgG (ascites) conjugate formed is centrifuged at 1300 x g (4°C) for 5 minutes. The supernatant is collected on a suction filter, and the loose pellet consisting of the conjugate is re-suspended in 500 $\mu$l 5 mM NaCl solution.

Testing procedure and Results

Nitrocellulose test strips of 1.2 x 5.5 cm are cut from nitrocellulose paper having a pore diameter of 0.45 $\mu$m. A concentration series of goat-anti-mouse polyclonal IgG (1280 ng - 2.5 pg) is spotted. As a negative control, a concentration series of $\beta$-galactosidase (1500 ng - 2.0 pg) is included.

Cross-incubation of the various strips is effected with both RITC/$SiO_2$-IgG (ascites) conjugate and with unprotected RITC/$SiO_2$ sol using a 1:50 dilution in PBS + 1% BSA for 2 hours at most. During incubation strips plus incubation mixture are agitated on a shaker in polypropylene tubes.

Goat-anti-mouse spots are specifically stained by the RITC/$SiO_2$-IgG (ascites) conjugate up to 40 ng. Goat-anti-mouse spots give no specific staining with unprotected RITC/$SiO_2$ sol.

$\beta$-galactosidase spots exhibit no specific staining whatsoever either with the RITC/$SiO_2$-IgG (ascites) conjugate or with unprotected RITC/$SiO_2$ sol.

## Claims

1. A method of determining in a test sample one or more components of the reaction between a specifically binding protein and the corresponding bindable substance, wherein use is made of the mutual reactivity of such components and of at least one labelled component obtained by coupling or adsorbing particles of a sol of the label directly or indirectly to the component, comprising during or after the completion of the reaction, optionally after the separation of the bound and the free labelled component, determining in said test sample, or in one of the fractions obtained after separation, the presence and/or the quantity of the label by a method suitable for the purpose to obtain a qualitative or quantitative indication of the component to be determined, characterized by using for the labelling a sol of a non-metallic element selected from the group consisting of elemental P, elemental C and elemental Si, or an inorganic compound of an element selected from the group consisting of S, Se, Te, P and Si, which inorganic compound does not contain metallic elements.

2. A method as claimed in claim 1, characterized in that the labelled component is obtained by adding to a sol of a non-metallic element selected from the group consisting of elemental P, elemental C and elemental Si, or an inorganic compound of an element selected from the group consisting of S, Se, Te, P and Si, which inorganic compound does not contain metallic elements, a pre-determined amount of the component to be labelled, said last component enveloping the so] particles in full or in part, and optionally followed by further envelopment with an inert polar macromolecule.

3. A method as claimed in claim 1, characterized in that the labelled component is obtained by adding to a sol of a non-metallic element selected from the group consisting of elemental P, elemental C and elemental Si, or an inorganic compound of an element selected from the group consisting of S, Se, Te, P and Si, which inorganic compound does not contain metallic elements, one or more inert hydrophilic macromolecules which envelop the nonmetallic particles, whereafter the component is coupled to the enveloping material by adsorption or via covalent bonds.

4. A method as claimed in claim 1, characterized in that the labelled compound is obtained by placing a sol of a non-metallic element selected from the group consisting of elemental P, elemental C and elemental Si, or an inorganic compound of an element selected from the group consisting of S, Se, Te, P and Si, which inorganic compound does not contain metallic elements, in a medium of monomers, causing said monomers to polymerize or copolymerize in situ, whereby envelopment of the sol particles of the non-metallic element or inorganic compound thereof, which does not contain metallic elements, follows, and then adsorbing or covalently coupling the component to the polymeric material.

5. A method as claimed in claim 4, characterized in that the sol particles of a non-metallic element selected from the group consisting of elemental P, elemental C and elemental Si, or an inorganic compound of an element selected from the group consisting of S, Se, Te, P and Si, which inorganic compound does not contain metallic elements, are first protected by a hydrophilic macromolecule, whereafter (co)-polymerization takes place by means of an inorganic initiator.

6. A method as claimed in any of the preceding claims, characterized by using as the sol particles elemental phosphorus, carbon and/or silicon.

7. A method as claimed in any of the preceding claims, characterized by using as the sol particles silicon dioxide.

8. A method as claimed in any of the preceding claims, characterized in that the component(s) to be determined consist(s) of one or more receptor proteins and/or antigenic determinants present at the surface of cells.

9. A method as claimed in any of the preceding claims, characterized in that the component(s) to be determined consist(s) of one or more immunological components, such as haptens, antigens or antibodies, present in an aqueous test sample.

10. A method of preparing a labelled component of the reaction between a specifically binding protein and the corresponding bindable substance by coupling or adsorbing particles of a sol of the label directly or indirectly to the component, characterized by using for the labelling a sol of a non-metallic element selected from the group consisting of elemental P, elemental C and elemental Si, or an inorganic compound of an element selected from the group consisting of S, Se, Te, P and Si, which inorganic compound does not contain metallic elements.

11. A method as claimed in claim 10, characterized by using for the labelling a sol of particles of elemental phosphorus, carbon and/or silicon, or silicon dioxide.

12. A test kit for use in determining an immunocomponent in an aqueous test sample, comprising
    (a) a labelled immunocomponent containing as the label sol particles of a non-metallic element selected from the group consisting of elemental P, elemental C and elemental Si, or an inorganic compound of an element selected from the group consisting of S, Se, Te, P and Si, which inorganic compound does not contain metallic elements; and
    (b) other reagents.

13. Labelled material consisting of an immunocomponent (for example, immunoglobulin, protein, hapten) or a different macromolecular organic material (for example, DNA, RNA) containing as a label sol particles of a non-metallic element selected from the group consisting of elemental P, elemental C and elemental Si, or an inorganic compound of an element selected from the group consisting of S, Se, Te, P and Si, which inorganic compound does not contain metallic elements.

**Patentansprüche**

1. Verfahren zur Bestimmung einer oder mehrerer Komponenten der Reaktion zwischen einem spezifisch bindenden Protein und der entsprechenden bindungsfähigen Substanz in einer Probe, wobei die gegenseitige Reaktivität derartiger Komponenten und mindestens einer markierten Komponente ausgenutzt wird, die durch Kupplung oder Adsorption von Teilchen eines Sols der Markierung direkt oder indirekt an die Komponente erhalten wurde, wobei das Verfahren während oder nach dem Abschluß der Reaktion und wahlweise nach der Trennung der gebundenen und der freien markierten Komponente die Bestimmung der Gegenwart und/oder der Menge der Markierung in der Probe oder in einer der nach der Trennung erhaltenen Fraktionen durch eine zu dem Zweck geeignete Methode, eine qualitative oder quantitative Anzeige der zu bestimmenden Komponente zu erhalten, umfaßt, dadurch gekennzeichnet, daß zur Markierung das Sol eines nicht-metallischen Elements, das unter elementarem P, elementarem C und elementarem Si ausgewählt ist, oder einer anorganischen Verbindung eines Elements, das unter S, Se, Te, P und Si ausgewählt ist, wobei die anorganische Verbindung keine metallischen Elemente enthält, verwendet wird.

2. Verfahren nach Anspruch 1, dadurch gekennzeichnet, daß die markierte Komponente durch Zugabe eines Sols eines nicht-metallischen Elements, das unter elementarem P, elementarem C und elementarem Si ausgewählt ist, oder einer anorganischen Verbindung eines Elements, das unter S, Se, Te, P und Si ausgewählt ist, wobei die anorganische Verbindung keine metallischen Elemente enthält, zu einer zuvor bestimmten Menge der zu markierenden Komponente, wobei letztere Komponente die Solteilchen ganz oder teilweise umhüllt, und wahlweise weitere Umhüllung mit einem inerten polaren Makromolekül erhalten wird.

3. Verfahren nach Anspruch 1, dadurch gekennzeichnet, daß die markierte Komponente durch Zugabe eines Sols eines nicht-metallischen Elements, das unter elementarem P, elementarem C und elementarem Si ausgewählt ist, oder einer anorganischen Verbindung eines Elements, das unter S, Se, Te, P und Si ausgewählt ist, wobei die anorganische Verbindung keine metallischen Elemente enthält, zu einem oder mehreren inerten hydrophilen Makromolekülen, die die nicht-metallischen Teilchen umhüllen, wobei danach die Komponente durch Adsorption oder über kovalente Bindungen an das umhüllende Material gekuppelt wird, erhalten wird.

4. Verfahren nach Anspruch 1, dadurch gekennzeichnet, daß die markierte Verbindung erhalten wird, indem man ein Sol eines nicht-metallischen Elements, das unter elementarem P, elementarem C und elementarem Si ausgewählt ist, oder einer anorganischen Verbindung eines Elements, das unter S, Se, Te, P und Si ausgewählt ist, wobei die anorganische Verbindung keine metallischen Elemente enthält, in ein Medium aus Monomeren gibt, bewirkt, daß die Monomeren in situ polymerisieren oder copolymerisieren, wobei die Solteilchen des nicht-metallischen Elements oder der anorganischen Verbindung davon, die keine metallischen Elemente enthält, umhüllt werden, und anschließend die Komponenten an das polymere Material adsorbiert oder kuppelt.

5. Verfahren nach Anspruch 4, dadurch gekennzeichnet, daß die Solteilchen des nicht-metallischen Elements, das unter elementarem P, elementarem C und elementarem Si ausgewählt ist, oder einer anorganischen Verbindung eines Elements, das unter S, Se, Te, P und Si ausgewählt ist, wobei die anorganische Verbindung keine metallischen Elemente enthält, zuerst durch ein hydrophiles Makromolekül geschützt werden, wobei danach eine (Co-)Polymerisation mittels eines anorganischen Initiators stattfindet.

6. Verfahren nach einem der vorstehenden Ansprüche, gekennzeichnet durch die Verwendung von elementarem Phosphor, Kohlenstoff und/oder Silicium als Solteilchen.

7. Verfahren nach einem der vorstehenden Ansprüche, gekennzeichnet durch die Verwendung von Siliciumdioxid als Solteilchen.

8. Verfahren nach einem der vorstehenden Ansprüche, dadurch gekennzeichnet, daß die zu bestimmende(n) Komponente(n) aus einem oder mehreren Rezeptorproteinen und/oder antigenen Determinanten, die an der Oberfläche von Zellen vorhanden sind, bestehen.

9. Verfahren nach einem der vorstehenden Ansprüche, dadurch gekennzeichnet, daß die zu bestimmende(n) Komponente(n) aus einem oder mehreren immunologischen Komponenten, wie Haptenen, Antigenen oder Antikörpern, die in einer wäßrigen Probe vorhanden sind, bestehen.

10. Verfahren zur Herstellung einer markierten Komponente der Reaktion zwischen einem spezifisch bindenden Protein und der entsprechenden bindungsfähigen Substanz durch Kupplung oder Adsorption von Teilchen eines Sols der Markierung direkt oder indirekt an die Komponente, gekennzeichnet durch die Verwendung eines Sols eines nichtmetallischen Elements, das unter elementarem P, elementarem C und elementarem Si ausgewählt ist, oder einer anorganischen Verbindung eines Elements, das unter S, Se, Te, P und Si ausgewählt ist, wobei die anorganische Verbindung keine metallischen Elemente enthält, zur Markierung.

11. Verfahren nach Anspruch 10, gekennzeichnet durch die Verwendung eines Sols von Teilchen von elementarem Phosphor, Kohlenstoff und/oder Silicium oder Siliciumdioxid zur Markierung.

12. Reagenzsatz zur Verwendung bei der Bestimmung einer Immunkomponente in einer wäßrigen Probe, umfassend

(a) eine markierte Immunkomponente, die als Markierung Solteilchen eines nicht-metallischen Elements, das unter elementarem P, elementarem C und elementarem Si ausgewählt ist, oder einer anorganischen Verbindung eines Elements, das unter S, Se, Te, P und Si ausgewählt ist, wobei die anorganische Verbindung keine metallischen Elemente enthält, enthält; und

(b) weitere Reagenzien.

13. Markiertes Material, bestehend aus einer Immunkomponente (z.B. Immunglobulin, Protein, Hapten) oder einem anderen makromolekularen organischen Material (z.B. DNA, RNA), enthaltend als Markierung Solteilchen eines nicht-metallischen Elements, das unter elementarem P, elementarem C und elementarem Si ausgewählt ist, oder einer anorganischen Verbindung eines Elements, das unter S, Se, Te, P und Si ausgewählt ist, wobei die anorganische Verbindung keine metallischen Elemente enthält.

**Revendications**

1. Procédé de détermination, dans un échantillon pour essais, d'un ou plusieurs composants de la réaction entre une protéine à liaison spécifique et la substance à lier correspondante, faisant usage de la réactivité mutuelle de tels composants et d'au moins un composant marqué obtenu en couplant ou en adsorbant des particules d'un sol de marqueur directement ou indirectement au composant, comprenant pendant ou après l'achèvement de la réaction, facultativement après la séparation du composant lié et du composant marqué libre, la détermination dans ledit échantillon pour essais ou dans une des fractions obtenues après séparation, de la présence et/ou de la quantité du marqueur par un procédé convenable dans le but d'obtenir une indication qualitative ou quantitative du composant à déterminer, caractérisé en ce que l'on utilise pour le marquage un sol d'un élément non-métallique choisi à partir du groupe constitué de l'élément P, de l'élément C et de l'élément Si, ou d'un composé minéral d'un élément choisi à partir du groupe constitué de S, Se, Te, P et Si, ledit composé minéral ne contenant pas d'éléments métalliques.

2. Procédé selon la revendication 1, caractérisé en ce que le composant marqué est obtenu en ajoutant à un sol d'un élément non-métallique choisi à partir du groupe constitué de l'élément P, de l'élément C et de l'élément Si, ou d'un composé minéral d'un élément choisi à partir du groupe constitué de S, Se, Te, P et Si, ledit composé minéral ne contenant pas d'éléments métalliques, une quantité prédéterminée du composant à marquer, ledit dernier composant enrobant entièrement ou en partie les particules en solution, et se trouve facultativement suivi par un enrobage ultérieur au moyen d'un macromolécule polaire inerte.

3. Procédé selon la revendication 1, caractérisé en ce que le composant marqué est obtenu en ajoutant à un sol d'un élément non-métallique choisi à partir du groupe constitué de l'élément P, de l'élément C et de l'élément Si, ou d'un composé minéral d'un élément choisi à partir du groupe constitué de S, Se, Te, P et Si, ledit composé minéral ne contenant pas d'éléments métalliques, une ou plusieurs macromolécules hydrophiles inertes qui enrobent les particules non-métalliques, après quoi le composant est couplé au matériau enrobant par adsorption ou par l'intermédiaire de liaisons covalentes.

4. Procédé selon la revendication 1, caractérisé en ce que le composant marqué est obtenu en plaçant un sol d'un élément non-métallique choisi à partir du groupe constitué de l'élément P, de l'élément C et de l'élément Si, ou d'un composé minéral d'un élément choisi à partir du groupe constitué de S, Se, Te, P et Si, ledit composé minéral ne contenant pas d'éléments métalliques, dans un milieu de monomères, en entraînant la polymérisation ou copolymérisation desdits monomères *in situ* à la suite de quoi se produit l'enrobage des particules du sol de l'élément métallique ou de son composé minéral qui ne contient pas d'éléments métalliques, et ensuite en provoquant l'adsorption ou la liaison covalente du composant à la matière polymère.

5. Procédé selon la revendication 4, caractérisé en ce que les particules du sol d'un élément non-métallique choisi à partir du groupe constitué de l'élément P, de l'élément C et de l'élément Si, ou d'un composé minéral d'un élément choisi à partir du groupe constitué de S, Se, Te, P et Si, ledit composé minéral ne contenant pas d'éléments métalliques, sont d'abord protégées par une macromolécule hydrophile, à la suite de quoi la (co)-polymérisation se produit au moyen d'un initiateur minéral.

6. Procédé selon l'une quelconque des revendications précédentes, caractérisé en ce que l'on utilise, comme particules du sol, les éléments phosphore, carbone et/ou silicium.

7. Procédé selon l'une quelconque des revendications précédentes, caractérisé en ce que l'on utilise, comme particules de sol, le dioxyde de silicium.

8. Procédé selon l'une quelconque des revendications précédentes, caractérisé en ce que le(s) composant(s) à déterminer consiste(nt) en une ou plusieurs protéines réceptives et/ou des déterminants antigéniques présents à la surface des cellules.

9. Procédé selon l'une quelconque des revendications précédentes, caractérisé en ce que le(s) composant(s) à déterminer consiste(nt) en un ou plusieurs composants immunologiques, tels que haptènes, antigènes ou anticorps, présents dans un échantillon aqueux pour essais.

10. Procédé de préparation d'un composant marqué de la réaction entre une protéine à liaison spécifique et la substance à lier correspondante, en couplant ou adsorbant les particules d'un sol du marqueur directement ou indirectement au composant, caractérisé en ce que l'on utilise pour le marquage un sol d'un élément non-métallique choisi à partir du groupe constitué de l'élément P, de l'élément C et de l'élément Si, ou d'un composé minéral d'un élément choisi à partir du groupe constitué de S, Se, Te, P et Si, ledit composé minéral ne contenant pas d'éléments métalliques.

11. Procédé selon la revendication 10, caractérisé en ce qu'on utilise pour le marquage un sol de particules des éléments phosphore, carbone et/ou silicium, ou du dioxyde de silicium.

12. Nécessaire au kit d'essais utilisé pour la détermination d'un immunocomposant dans un échantillon aqueux pour essais, comprenant :

(a) un immunocomposant marqué contenant comme marqueur des particules du sol d'un élément non-métallique choisi à partir du groupe constitué de l'élément P, de l'élément C et de l'élément Si, ou d'un composé minéral d'un élément choisi à partir du groupe constitué de S, Se, Te, P et Si, ledit composé minéral ne contenant pas d'éléments métalliques,
(b) d'autres réactifs.

13. Matière marquée constituée d'un immunocomposant (par exemple, immunoglobuline, protéine, haptène) ou d'une matière organique macromoléculaire différente (par exemple, l'ADN ou l'ARN) contenant comme marqueur des particules d'un sol d'un élément non-métallique choisi à partir du groupe constitué de l'élément P, de l'élément C et de l'élément Si, ou d'un composé minéral d'un élément choisi à partir du groupe constitué de S, Se, Te, P et Si, ledit composé minéral ne contenant pas d'éléments métalliques.